# EUROPEAN PATENT APPLICATION

(11) **EP 4 763 830 A1**
(43) Date of publication of application: **24.06.2026**
(21) Application number: 24222874.0
(22) Date of filing: 23.12.2024
(51) Int. Cl.: C07C 209/62, C07C 211/46, C07C 211/45, C07C 211/50, C08J 11/04

(54) **METHOD FOR PREPARING AMINE COMPOUNDS**

(71) Applicant: École Polytechnique Fédérale de Lausanne (EPFL), 1015 Lausanne (CH)
(72) Inventor: Wu, Xinbang, 1024 Ecublens (CH); Dyson, Paul, 1024 Ecublens (CH)
(74) Representative: Cohausz & Florack

(57) **Abstract**

Herein described is a method for manufacturing an amine, in particular an aromatic amine, from a carbamate compound, in particular an aromatic carbamate compound, comprising a cleaving step, in which the aromatic carbamate compound is heated to a temperature of 50°C to 500°C in the presence of a reducing agent and in the presence of a catalyst, wherein the catalyst has Lewis acidity and comprises a metal.

## Description

### TECHNICAL FIELD

The invention relates to a method for manufacturing an amine from a carbamate compound.

### BACKGROUND

The proliferation of plastic waste poses an increasingly pressing environmental challenge for the near future. Polyurethanes (PU) represent a type of plastic derived from the addition polymerization of diisocyanates and polyols. PUs are renowned for their versatility and can be modified by selecting different monomer fractions to create a wide array of elastomers, thermoplastics, flexible and rigid foams, fibres, adhesives, and coatings. Methylene diphenyl diisocyanate (2,4-MDI or 2,6-MDI) and toluene diisocyanate (2,4-TDI or 2,6-TDI) are the predominant isocyanate monomers used for PU synthesis, due to their lower volatility which allows for safer handling. These monomers are industrially manufactured from the phosgenation of their respective diamines, methylene diphenyl diamine (MDA) and toluene diamine (TDA), which are sourced from commodity feedstocks such as aniline. Therefore, there is an incentive to explore possible PU recycling pathways to recover these compounds and reduce the reliance on fossil resources for PU production. Furthermore, the high toxicity of these chemicals poses concern during degradation of PU in the environment, highlighting the importance of implementing appropriate end-of-life treatment.

Chemical recycling of PU has been previously studied, mainly via solvolysis, aminolysis, and glycolysis (e.g.: Mahoney, L. R., Weiner, S. A., Ferris, F. C., Environ. Sci. Technol. 1974, 8, 135-139; Johansen, M. B., Donslund, B. S., Henriksen, M. L., Kristensen, S. K., Skrydstrup, T., Green Chem. 2023, 25, 10622-10629; Grdadolnik, M. et al., ACS Sustainable Chem. Eng. 2023, 11, 10864-10873; Zahedifar, P., Pazdur, L., Van de Velde, C. M. L., Billen, P., Sustainability 2021, 13**;** Simón, D., Borreguero, A. M., de Lucas, A., Rodriguez, J. F., Waste Manage. 2018, 76, 147-171). Although isocyanates are the usual PU monomers, obtaining high yields of their amine derivatives, which can be readily converted to isocyanates, could also be valuable for producing new polymers or other compounds. Nevertheless, the hydrogenation methods typically employed for conversion to the amine derivatives necessitate the use of a base and homogeneous catalysts, making separation of the catalyst and products challenging, thereby diminishing the overall sustainability of the approach. Ideally, the depolymerization of PU towards its amine derivatives could be conducted in a system that utilizes an inexpensive catalyst that is recoverable and reusable.

Thus, it is an object of the present invention to provide a method that addresses these and other needs in the state of the art by providing an improved reaction system for the preparation of amine compounds from carbamate compounds, such as for example PU compounds.

Other and further objects, features and advantages of the present invention will become apparent more fully from the following description.

### SUMMARY OF THE INVENTION

Some or all of these objects are achieved with the present invention by the method according to the invention. The method according to the invention is a method for manufacturing an amine, in particular an aromatic amine, from a carbamate compound, in particular an aromatic carbamate compound, comprising a cleaving step, in which the aromatic carbamate compound is heated to a temperature of 50 °C to 500 °C in the presence of a reducing agent and in the presence of a catalyst, wherein the catalyst has Lewis acidity and comprises a metal.

It was surprisingly found that, employing the method according to the invention, near-quantitative conversions of carbamate compounds could be achieved with a good yield of amine products. Some of the amine products obtained through the method according to the invention can be readily converted back into isocyanate monomers via phosgenation, thus providing a circular route for waste PU recycling. Further, it was found that not only hydrogen can be used as a reducing agent, but also, for example, hydrogen donors such as water or alcohols.

Without wishing to be bound by scientific theory, it is believed that certain characteristics of the catalyst, such as Lewis acidity, ability to form surface oxygen vacancies, and redox activity, promote carbamate bond dissociation and the production of amine compounds.

The term "Lewis acidity" may in particular refer to the thermodynamic tendency of a substrate to act as a Lewis acid. A Lewis acid is in particular a chemical species that contains an empty orbital which is capable of accepting an electron pair, in particular from a Lewis base, thereby forming a Lewis adduct. Lewis acidity is in particular the ability to accept an electron pair. A compound, for example a molecular compound, may comprise Lewis acidity. A substrate, such as the surface of a particle, may comprise Lewis acidity. In particular, a substrate, such as a particle, may comprise sites and/or entities that are capable of accepting an electron pair from another compound, for example a Lewis base. On a substrate, such as a particle, sites comprising Lewis acidity may for example be sites that are deficient of a surface atom, such as a hydroxide layer with one or more hydroxyl vacancies or an oxide layer with one or more oxygen vacancies. Comparative measures of Lewis acidity may be provided by the equilibrium constants for Lewis adduct formation of a series of Lewis acids with a common reference Lewis base. Simple Lewis acids are those that react directly with the Lewis base, while complex Lewis acids require an activation step prior to formation of the adduct with the Lewis base.

Preferably, the metal comprised in the catalyst may comprise Lewis acidity.

### PREFERRED EMBODIMENTS OF THE INVENTION

Further advantageous embodiments of the invention are specified in the dependent claims and are elucidated in detail herein below.

According to a preferred embodiment of the method according to the invention, the amine is selected from the group consisting of aliphatic amines, aromatic amines, and mixtures thereof. In particular, the amines are derivatives of isocyanates, preferably the amine obtained from an isocyanate by reaction with water. Advantageously, the amine is selected from the group consisting of hexamethylenediamine, isophorone diamine, cyclohexylamine, aminotoluene, aniline, ethylaniline, benzyl aniline, diaminotoluene, methylene diphenyl diamine, polymeric methylene diphenyl diamine, tetramethylxylylene diamine, and mixtures thereof. Preferably, the amine is selected from the group consisting of aminotoluene, aniline, ethylaniline, benzyl aniline, diaminotoluene, methylene diphenyl diamine, polymeric methylene diphenyl diamine, tetramethylxylylene diamine, and mixtures thereof. For example, aromatic amines such as aniline, methylene diphenyl diamine, and toluene diamine can be used to synthesize isocyanate monomers for the manufacture of PU but may also be used to synthesize other polymers or other compounds.

In a preferred embodiment of the method according to the invention, the carbamate compound is a non-polymeric compound or a polymeric compound, in particular a polyurethane. Examples of polyurethanes include thermoplastic polyurethanes, thermoset polyurethanes. The polyurethane may in particular be a waste polyurethan. The polyurethane may be in different forms such as a workpiece, granules, or foams. With the method according to the invention, it is even possible to convert commercial PU into its amine derivatives. The method was demonstrated to be suitable for various commercial PU products, including a flexible foam used as a stopper, a rigid foam used as insulation (Tricast-5), and a common washing sponge.

Advantageously, the cleaving step of the method according to the invention is conducted for a period of 15 min to 48 h, preferably 30 min to 24 h, more preferably 45 min to 12 h, most preferably 1 h to 5 h. For reaction times of this duration, a particularly high yield of the target products is obtained, while longer reaction times may result in a reduction in amine yield due to side reactions.

According to an embodiment of the method according to the invention, the metal comprised in the catalyst is selected from the group consisting of aluminum, zirconium, silicon, titanium, niobium, lanthanum, cerium, iron, copper, vanadium, molybdenum, tin, indium, cadmium, rhodium, antimony, germanium, zinc, bismuth, gallium, and mixtures thereof. Preferably, the metal is selected from the group consisting of aluminum, lanthanum, cerium and mixtures thereof. More preferably, the metal comprised in the catalyst is cerium. Advantageously, the catalyst is a metal oxide. The term "metal oxide" may in particular refer to a binary compound composed of one or more oxygen atoms and one or more metal atoms. Metal-oxide catalysts are particularly suitable for industrial application due to their high abundance and low cost. Despite having limited hydrogenation ability compared to noble metal catalysts, metal oxides have unique interactions with hydrogen.

Advantageously, the catalyst used in the method according to the invention is a heterogeneous catalyst. Heterogeneous catalysts have several advantages over homogeneous catalysts, such as reusability, easier separation of product and catalyst, often lower costs, and greater robustness to severe reaction conditions. The catalyst is preferably in the form of particles, more preferably nanoparticles. The size of the particles is preferably 300 nm or lower, more preferably 100 nm or lower, most preferably 50 nm or lower. The catalyst preferably has a specific surface area of 10 m²/g or more, more preferably of 20 m²/g or more, even more preferably of 30 m²/g or more, most preferably of 40 m²/g or more. The specific surface area may be determined according to ISO 9277:2010.

In a preferred embodiment of the method according to the invention, the catalyst is selected from the group consisting of titanium dioxide, zirconium dioxide, niobium pentoxide, aluminum cerium trioxide, lanthanum oxide, cerium dioxide, γ-aluminum oxide, Y-zeolite, ZSM-5 zeolite, vanadium pentoxide, cuprous oxide, molybdenum oxide, stannic oxide, diindium trioxide, rhodium oxide, diantimony trioxide, dibismuth trioxide, germanium dioxide, zinc oxide, gallium oxide, and mixtures thereof. Preferably, the catalyst is selected from the group consisting of aluminum cerium trioxide, lanthanum oxide, cerium dioxide, γ-aluminum oxide, Y-zeolite, and mixtures thereof. More preferably, the catalyst is cerium dioxide. Using the above catalysts, high conversion of the carbamate compound was achieved. Without wishing to be bound by scientific theory, it is believed that a high Lewis acidity is particularly beneficial for achieving high conversion of the carbamate compound as well as high yield of the amine compound. The remarkable activity of CeOz is believed to be attributable to its combination of high Lewis acidity, which promotes hydrogen dissociation, highly redox active Ce³⁺/Ce⁴⁺ pairs, and low formation energy of surface oxygen vacancies, which promotes the production of amines and COz from carbamate compounds via a Mars-van Krevelen mechanism.

Advantageously, the carbamate compound is heated in the cleaving step to a temperature of 80 °C to 400°C, preferably 100 °C to 300 °C, more preferably 150 °C to 250 °C. At these temperatures, high conversion of carbamate compounds is achieved while retaining good selectivity to the desired amine compounds.

According to an embodiment of the method according to the invention, the reducing agent is selected from the group consisting of hydrogen, sodium hydride, sodium borohydride, lithium aluminum hydride, hydrazine, formic acid, hydrogen donors such as water, alcohols, in particular methanol, ethanol, isopropanol or glycerol, and saturated alkanes such as polyethylene and mixtures thereof. Preferably, the reducing agent is hydrogen, in particular hydrogen at a pressure from 1 to 200 bar, preferably from 1 to 150 bar, more preferably from 5 to 100 bar, even more preferably from 5 to 50 bar, most preferably from 8 to 30 bar. The use of hydrogen donors such as water, alcohols, and saturated alkanes can serve as an alternative, hydrogen-free pathway for carbamate bond cleavage, while the use of hydrogen as reducing agent is particularly suitable for an industrial process. In particular when using alcohol as a hydrogen donor, the alcohol may at the same time serve as a solvent. When using alcohol as a hydrogen donor, the method may be conducted in air or under an inert gas atmosphere. Examples for inert gases are nitrogen, argon, and/or xenon, in particular nitrogen and/or argon.

In a preferred embodiment of the method according to the invention, the cleaving step is conducted in the absence of a solvent. This has both economic and ecological advantages as no solvent costs need to be added to the production costs, and no solvent waste is generated. Further, the product does not need to be separated from the solvent after the reaction.

According to a preferred embodiment of the method according to the invention, the catalyst is present in an amount of 0.01 wt.% to 20 wt.%, preferably 0.1 wt.% to 15 wt.%, more preferably 1 wt.% to 10 wt.%, based on the mass of the carbamate compound. By employing a catalyst amount in this range, a sufficient amount of catalyst is present to achieve high conversion of the carbamate compounds without incurring unnecessary costs by using an unnecessarily high amount of catalyst.

Advantageously, the method according to the invention comprises an activation step in which the catalyst is heated to a temperature of 300 °C or more, preferably from 300 °C to 1000 °C, more preferably from 400°C to 800 °C. This activation step can be performed under air or under inert gas atmosphere, such as nitrogen and/or argon atmosphere. Catalysts activated under the described conditions achieved a particularly high carbamate conversion and amine yield. Without wishing to be bound by scientific theory, it is believed that activation of the catalyst at elevated temperatures of 300 °C or more, in particular under inert gas atmosphere, leads to the formation of oxygen vacancies, which are believed to play a significant role in the synthesis of the amine compounds.

In a further embodiment of the method according to the invention, the method also produces alcohols and/or polyols. For example, compounds such as ethylene glycol, 1-butanol, and ethanol could be produced starting from phenylurethane, ethane-1,2-diylbis((4-benzylphenyl)carbamate), and dibutyl(methylenebis(4,1-phenylene))dicarbamate, respectively.

In the following, the invention is further described by way of examples that are in no way meant to be limiting.

### EXAMPLES

### Materials

Cerium(IV) oxide, aluminium cerium(III) oxide, niobium(V) pentoxide, acidic gamma-alumina oxide, methylenediphenyl-4,4'-diisocyanate, 1-butanol, 4-benzylaniline, ethylenebis(chloroformate) and dibutyltindilaurate were purchased from Sigma-Aldrich. Phenylurethane was purchased from TCI. Zirconium(IV) oxide, silicon dioxide, Y-zeolite and ZSM-5 zeolite were purchased from acbr GmbH. Titanium(IV) oxide was purchased from Fluka. Lanthanum(III) oxide was purchased from Strem Chemicals. Thermoplastic polyurethane granules and rigid polyurethane foam Tricast-5 were purchased from Goodfellow. Polyurethane foam stoppers were purchased from Thermo Fisher.

The following compounds were used as model carbamate compounds:
M1: phenylurethane
M2: ethane-1,2-diylbis((4-benzylphenyl)carbamate)
M3: dibutyl(methylenebis(4,1-phenylene))dicarbamate

### Synthesis of M2

To a solution of degassed and dried MeCN (50 mL), 4-benzylaniline (9.16 g, 50 mmol) and potassium carbonate (10.0 g, 72.4 mmol) was added, followed by stirring for 5 min in an ice bath. Next, ethylenebis(chloroformate) (5.61 g, 30 mmol) was added dropwise under vigorous stirring and the resulting mixture was stirred for 3 h. The crude product was filtered and washed twice with water and twice with ethanol, resulting in a white powdery solid. ¹H NMR (400 MHz, (CD₃)₂SO) δ 9.66 (s, 2H), 7.38-7.36 (d, 4H), 7.28-7.25 (m, 4H), 7.19-7.10 (m, 10H), 4.30 (s, 4H), 3.85 ppm (s, 4H). Melting point: 178°C.

### Synthesis of M3

To a solution of degassed and dried 1-butanol (50 mL), methylenediphenyl-4,4'-diisocyanate (MDI) (9.16 g, 50 mmol) was added. Dibutyltindilaurate (10 µL) was then added to the solution under vigorous stirring. The mixture is heated up to 50°C and stirred for 3 h. The crude product was filtered and washed twice with water and twice with ethanol, resulting in a white powdery solid. ¹H NMR (400 MHz, (CD₃)₂SO) δ 9.49 (s, 2H), 7.36-7.33 (d, 4H), 7.09-7.07 (d, 4H), 4.07-4.03 (t, 4H), 3.78 (s, 2H), 1.62-1.55 (m, 4H), 1.41-1.32 (m, 4H), 0.92-0.89 ppm (t, 6H). Melting point: 121°C.

### Analytical methods

### Product analysis for model compounds

The glass-vial was removed from the autoclave and the products were extracted with ether (M1) or methanol (M2 and M3). Solid residues were separated from the mixture via centrifugation and air-dried. The liquid fraction was analysed using GC-MS on an Agilent 7000 C instrument using an Agilent DB-17 (50%-Phenyl)- methylpolysiloxane column with hexadecane as an internal standard. The conversion of M1 was determined from the intensity of its GC-MS signal using a calibration curve. The conversion of M2 and M3 was determined by subtracting the mass of the catalyst from the mass of the solid residue, yielding the mass of insoluble products, which was assumed to consist only of unreacted substrate.

### Product analysis for commercial PU

The glass-vial was removed from the autoclave and the products were extracted with ether. Solid residues, containing catalyst and unreacted polymer, were separated from the mixture via centrifugation. The liquid fraction was separated via flash column chromatography using pentane/ethyl acetate (1:1) eluent, with a gradual increase of polarity to extract the aniline fraction. Lastly, methanol was used to extract the polyol fraction. The solvent was removed on a rotary-evaporator and the products weighed and analyzed by ¹H NMR spectroscopy after dissolution of a portion of the products in 1 mL D-chloroform. ¹H NMR spectroscopy measurements was performed on a Bruker Avance 400 MHz Spectrometer. The conversion of the PU was determined by subtracting the mass of the catalyst from the mass of the solid residue, yielding the mass of insoluble products, which was assumed to consist only of unreacted polymer.

### General procedure hydrogenation method

25 mg (50 mg) of catalyst and 250 mg of a model compound (M1, M2, or M3) or 500 mg of a PU sample was added to a pre-weighed glass vial with a stir-bar. The vial was transferred into a stainless-steel Parr autoclave (volume 75 mL) and was tightly sealed. The autoclave was pressurized to 10 bar of hydrogen and underwent five purge cycles. The autoclave was placed in a pre-heated Parr ceramic heater at 200 °C with stirring maintained at 700 rpm. At the end of the reaction, the autoclave was placed under running water in a water-bath until cooled.

### General procedure transfer-hydrogenation method

25 mg (50 mg) of catalyst, 250 mg of model compound (M1, M2, or M3) or 500 mg of a PU sample and 2 mL (5 mL for PU) of ethanol was added to a pre-weighed glass vial with a stir-bar. The vial was transferred into a stainless-steel Parr autoclave (volume 75 mL) and was tightly sealed. The autoclave was placed in a pre-heated Parr ceramic heater at 160 °C with stirring maintained at 700 rpm. At the end of the reaction, the autoclave was placed under running water in a water-bath until cooled.

### Catalyst screening

**Table 1: Conversion of M1 to aniline, phenylisocyanate, and COz.**

| **No.** | **Catalyst** | **Conv. [%]** | **Aniline yield [%]** | **Phenylisocyanate yield [%]** | **CO₂ yield [%]** |
|---|---|---|---|---|---|
| 1 | γ-Al₂O₃ | 100 | 80 | 0 | 9 |
| 2 | SiO₂ | 28 | 13 | 6 | 3 |
| 3 | ZSM-5 | 63 | 36 | 2 | 6 |
| 4 | Y-zeolite | 70 | 20 | 8 | 6 |
| 5 | TiO₂ | 49 | 40 | 3 | 5 |
| 6 | ZrO₂ | 34 | 5 | 12 | 3 |
| 7 | Nb₂O₅ | 58 | 33 | 2 | 5 |
| 8 | CeO₂ | 100 | 92 | 0 | 11 |
| 9 | AlCeO₃ | 95 | 80 | 0 | 11 |
| 10 | La₂O₃ | 81 | 74 | 0 | 9 |
| 11 | - | 19 | 7 | 9 | 2 |

| | | | | | |
|---|---|---|---|---|---|
| Reaction conditions: M1 (250 mg), catalyst (25 mg), Hz (10 bar), 200 °C, 2 h. | | | | | |

**Table 2: Conversion of M2 to 4-benzylaniline (4-BA), ethylene glycol, and COz.**

| **No.** | **Catalyst** | **Conv. [%]** | **4-BA yield [%]** | **Ethylene glycol yield [%]** | **CO₂ yield [%]** |
|---|---|---|---|---|---|
| 1 | γ-Al₂O₃ | 93 | 62 | 26 | 7 |
| 2 | SiO₂ | 24 | 9 | 16 | 4 |
| 3 | ZSM-5 | 69 | 18 | 13 | 3 |
| 4 | Y-zeolite | 92 | 18 | 13 | 2 |
| 5 | TiO₂ | 56 | 7 | 37 | 3 |
| 6 | ZrO₂ | 65 | 16 | 9 | 5 |
| 7 | Nb₂O₅ | 70 | 12 | 28 | 4 |
| 8 | CeO₂ | 100 | 90 | 22 | 6 |
| 9 | AlCeO₃ | 72 | 39 | 46 | 5 |
| 10 | La₂O₃ | 84 | 45 | 35 | 6 |
| 11 | - | 24 | 6 | 12 | 3 |

| | | | | | |
|---|---|---|---|---|---|
| Reaction conditions: M2 (250 mg), catalyst (25 mg), Hz (10 bar), 200 °C, 3.5 h. | | | | | |

**Table 3: Conversion of M3 to 2,4-methylene diphenyl diamine (2,4-MDA), 1-butanol, and COz.**

| **No.** | **Catalyst** | **Conv. [%]** | **2,4-MDA yield [%]** | **1-Butanol yield [%]** | **CO₂ yield [%]** |
|---|---|---|---|---|---|
| 1 | γ-Al₂O₃ | 71 | 38 | 70 | 6 |
| 2 | SiO₂ | 24 | 1 | 19 | 1 |
| 3 | ZSM-5 | 29 | 1 | 29 | 2 |
| 4 | Y-zeolite | 56 | 3 | 28 | 1 |
| 5 | TiO₂ | 29 | 3 | 29 | 3 |
| 6 | ZrO₂ | 52 | 3 | 34 | 2 |
| 7 | Nb₂O₅ | 36 | 1 | 36 | 2 |
| 8 | CeO₂ | 94 | 92 | 63 | 8 |
| 9 | AlCeO₃ | 46 | 6 | 46 | 3 |
| 10 | La₂O₃ | 70 | 36 | 69 | 3 |
| 11 | - | 23 | 1 | 23 | 2 |

| | | | | | |
|---|---|---|---|---|---|
| Reaction conditions: M3 (250 mg), catalyst (25 mg), Hz (10 bar), 200 °C, 2 h. | | | | | |

Tables 1 to 3 show the performance of various catalysts in the conversion of the three model carbamates M1, M2, and M3. While all tested catalysts showed significant activity, several catalysts - γ-AlzOa, CeOz, AlCeOs, and La₂O₃ - achieved particularly high conversions and yields, with CeOz showing the best performance among the tested catalysts. In comparison experiments without addition of a catalyst, some conversion took place, but the amine yield was very low.

### Reducing agents

**Table 4: Conversion of M1, M2, and M3 using water as reducing agent.**

| **No.** | **Reactant** | **Water [g]** | **p [bar]** | **t [h]** | **Conv. [%]** | **Amine yield [%]** | **Alcohol yield [%]** |
|---|---|---|---|---|---|---|---|
| 1 | M1 | - | 10 (H₂) | 2 | 100 | 92 | n.d. |
| 2 | M1 | 0.1 | 10 (H₂) | 2 | 98 | 72 | n.d. |
| 3 | M1 | 0.1 | 1 (N₂) | 2 | 100 | 92 | n.d. |
| 4 | M1 | 2 | 1 (N₂) | 2 | 75 | 60 | n.d. |
| 5 | M2 | - | 10 (H₂) | 3.5 | 100 | 90 | 22 |
| 6 | M2 | 0.1 | 10 (H₂) | 3.5 | 88 | 59 | 37 |
| 7 | M2 | 0.1 | 1 (N₂) | 3.5 | 94 | 57 | 28 |
| 8 | M2 | 2 | 1 (N₂) | 3.5 | 69 | 46 | 32 |
| 9 | M3 | - | 10 (H₂) | 2 | 94 | 92 | 63 |
| 10 | M3 | 0.1 | 10 (H₂) | 2 | 87 | 34 | 56 |
| 11 | M3 | 0.1 | 1 (N₂) | 2 | 97 | 93 | 88 |
| 12 | M3 | 2 | 1 (N₂) | 2 | 37 | 6 | 33 |

| | | | | | | | |
|---|---|---|---|---|---|---|---|
| Reaction conditions: model carbamate (250 mg), CeOz (25 mg), 200 °C; "n.d.": not determined. | | | | | | | |

**Table 5: Conversion of M1, M2, and M3 using alcohols as reducing agents.**

| **No.** | **Reactant** | **Alcohol** | **p [bar]** | **t [h]** | **Conv. [%]** | **Amine yield [%]** | **Alcohol yield [%]** |
|---|---|---|---|---|---|---|---|
| 1 | M1 | EtOH | 1 (air) | 24 | 33 | 13 | n.d. |
| 2 | M1 | iPrOH | 1 (air) | 24 | 96 | 73 | n.d. |
| 3 | M1 | MeOH | 1 (air) | 24 | 42 | 1 | n.d. |
| 4 | M1 | EtOH | 10 (N₂) | 24 | 33 | 22 | n.d. |
| 5^{a} | M1 | EtOH | 1 (air) | 24 | 1 | <1 | n.d. |
| 6 | M2 | EtOH | 1 (air) | 2 | 15 | 15 | 11 |
| 7 | M2 | EtOH | 1 (air) | 14 | 93 | 47 | 37 |
| 8 | M2 | EtOH | 1 (air) | 24 | 98 | 45 | 48 |
| 9 | M2 | iPrOH | 1 (air) | 24 | 99 | 42 | 48 |
| 10 | M2 | EtOH | 10 (N₂) | 24 | 95 | 49 | 76 |
| 11a | M2 | EtOH | 1 (air) | 24 | 92 | 2 | 35 |
| 12 | M3 | EtOH | 1 (air) | 24 | 74 | 24 | 74 |
| 13 | M3 | iPrOH | 1 (air) | 24 | 100 | 55 | 86 |
| 14 | M3 | EtOH | 10 (N₂) | 24 | 100 | 80 | 97 |
| 15a | M3 | EtOH | 1 (air) | 24 | 63 | <1 | 48 |

| | | | | | | | |
|---|---|---|---|---|---|---|---|
| Reaction conditions: model carbamate (250 mg), CeOz (25 mg), alcohol (2 mL), 160 °C; "n.d.": not determined.; ^{a} - no CeOz was used. | | | | | | | |

In addition to Hz, other reducing agents were tested in the conversion of the three model carbamates using CeOz as catalyst. Table 4 shows the effect of the addition of water on the reaction. While small amounts of water added to a Hz atmosphere had a slight negative effect on carbamate conversion and amine yield, the same amount of water added to an N₂ atmosphere resulted in conversions and yields comparable to those of the Hz system without addition of water.

Table 5 shows the results of using various alcohols as the reducing agent instead of Hz (i.e., in air or under N₂ atmosphere). Isopropanol in particular showed high conversions and yields. Further, reactions performed in the absence of a catalyst showed that the resulting amine yield was negligible (2% or less), while up to 80% of amine yield were achieved using CeOz as catalyst.

### Reaction time

**Table 6: Conversion of M1, M2, and M3 at various reaction times.**

| **No.** | **Reactant** | **t [h]** | **Conv. [%]** | **Amine yield [%]** | **Alcohol yield [%]** | **CO₂ yield [%]** |
|---|---|---|---|---|---|---|
| 1 | M1 | 1 | 88 | 39^{a} | n.d. | 7 |
| 2 | M1 | 1.5 | 98 | 68^{a} | n.d. | 10 |
| 3 | M1 | 2 | 100 | 92^{a} | n.d. | 11 |
| 4 | M1 | 5 | 100 | 62^{a} | n.d. | 11 |
| 5 | M2 | 2 | 81 | 60^{b} | 26^{c} | 5 |
| 6 | M2 | 3.5 | 100 | 90^{b} | 22^{c} | 6 |
| 7 | M2 | 7 | 100 | 75^{b} | 10^{c} | 6 |
| 8 | M2 | 16 | 100 | 62^{b} | 6^{c} | 6 |
| 9 | M3 | 2 | 94 | 92^{d} | 63^{e} | 8 |
| 10 | M3 | 2.5 | 100 | 99^{d} | 70^{e} | 8 |
| 11 | M3 | 3.5 | 100 | 95^{d} | 64^{e} | 9 |
| 12 | M3 | 5.5 | 100 | 22^{d} | 15^{e} | 9 |

| | | | | | | |
|---|---|---|---|---|---|---|
| Reaction conditions: model carbamate (250 mg), CeOz (25 mg), Hz (10 bar), 200 °C; "n.d.": not determined; ^{a} - amine = aniline; ^{b} - amine = 4-benzylaniline; ^{c} - alcohol = ethylene glycol; ^{d} - amine = 2,4-methylene diphenyl diamine; ^{e} - alcohol = 1-butanol. | | | | | | |

Table 6 shows the conversion of the three model carbamates at various reaction times. It can be seen that the amine yield reaches a peak and then decreases again at longer reaction times. Without wishing to be bound by scientific theory, it is believed that this is due to dimerization of the target amines at longer reaction times.

### Catalyst activation

**Table 7: Conversion of M1, M2, and M3 with CeOz activated at various temperatures.**

| **No.** | **Reactant** | **T_{activation} [°C]** | **t [h]** | **Conv. [%]** | **Amine yield [%]** | **Alcohol yield [%]** | **CO₂ yield [%]** |
|---|---|---|---|---|---|---|---|
| 1 | M1 | ^{_a} | 2 | 61 | 23 | n.d. | 5 |
| 2 | M1 | 300 | 2 | 84 | 25 | n.d. | 6 |
| 3 | M1 | 400 | 2 | 99 | 67 | n.d. | 10 |
| 4 | M1 | 500 | 2 | 100 | 89 | n.d. | 11 |
| 5 | M1 | 600 | 2 | 100 | 95 | n.d. | 11 |
| 6 | M2 | ^{_a} | 3.5 | 90 | 9 | 48 | 6 |
| 7 | M2 | 300 | 3.5 | 95 | 60 | 51 | 6 |
| 8 | M2 | 400 | 3.5 | 96 | 82 | 62 | 6 |
| 9 | M2 | 500 | 3.5 | 100 | 89 | 34 | 6 |
| 10 | M2 | 600 | 3.5 | 100 | 98 | 57 | 6 |
| 11 | M3 | ^{_a} | 2 | 83 | 54 | 50 | 8 |
| 12 | M3 | 300 | 2 | 98 | 68 | 60 | 8 |
| 13 | M3 | 400 | 2 | 98 | 73 | 28 | 8 |
| 14 | M3 | 500 | 2 | 100 | 78 | 65 | 8 |
| 15 | M3 | 600 | 2 | 100 | 100 | 89 | 7 |

| | | | | | | | |
|---|---|---|---|---|---|---|---|
| Reaction conditions: model carbamate (250 mg), CeOz (25 mg), Hz (10 bar), 200 °C; "n.d.": not determined; ^{a} - no activation step was performed. | | | | | | | |

The catalyst was optionally activated at different temperatures in an activation step. Table 7 shows the performance of CeOz activated under Ar atmosphere at 300, 400, 500, and 600 °C, respectively, compared with that of CeOz that was not activated. It can be seen that a higher activation temperature resulted in a marked increase in particular in amine yield. Without wishing to be bound by scientific theory, it is believed that activation of the catalyst at elevated temperatures leads to the formation of oxygen vacancies, which are believed to play a significant role in the synthesis of the amine compounds.

### Conversion of PU

**Table 8: Conversion of various PUs.**

| **No.** | **PU** | **t [h]** | **T [°C]** | **Conv. [%]** | **Amine yield [mg]** | **Alcohol yield [mg]** |
|---|---|---|---|---|---|---|
| 1^{a} | Thermoplastic granules | 8 | 200 | <1 | n.d. | n.d. |
| 2 | Thermoplastic granules | 8 | 200 | 87 | 146 (2,4-MDA) | 264 (butanediol-based polyol) |
| 3 | Thermoplastic granules | 24 | 200 | 100 | 162 (2,4-MDA) | n.d. |
| 4b | Thermoplastic granules | 8 | 200 | 73 | 68 (2,4-MDA) | n.d. |
| 5^{c} | Thermoplastic granules | 14 | 160 | 100 | 155 (2,4-MDA) | 343 (butanediol-based polyol) |
| 6 | Flexible foam | 8 | 200 | 79 | 86 (2,4-TDA + 2,6-TDA) | 302 (polyethylene glycol) |
| 7 | Washing sponge | 8 | 200 | 93 | 107 (2,4-TDA + 2,6-TDA) | 328 (unidentified polyol fraction) |
| 8 | Rigid foam | 8 | 200 | 8 | n.d. | n.d. |
| 9^{c} | Rigid foam | 14 | 160 | 55 | n.d. | n.d. |
| 10^{c} | Rigid foam | 14 | 180 | 97 | 97 (aniline + p-toluidine) | n.d. |

| | | | | | | |
|---|---|---|---|---|---|---|
| Reaction conditions: PU (500 mg), CeOz (50 mg), Hz (10 bar); "n.d.": not determined; 2,4-MDA = 2,4-methylene diphenyl diamine; 2,4-TDA = 2,4-diaminotoluene; 2,6-TDA = 2,6-diaminotoluene; ^{a} - no catalyst used; ^{b} - γ-AlzOa used as catalyst instead of CeOz; ^{c} - EtOH (5 mL) used as reducing agent instead of Hz. | | | | | | |

Table 8 shows the results of reactions employing various PUs as reactants and using CeOz as catalyst. Thermoplastic PU granules reacted well using both H₂ or EtOH as reducing agent, yielding 2,4-methylene diphenyl diamine as the amine product. A comparative experiment under catalyst-free conditions (Ex. 1) showed no significant conversion, while the use of γ-Al₂O₃ instead of CeOz resulted in a decrease in conversion and yield. The substrate scope was then extended to various other commercial PU products, including a flexible foam used as a stopper, a rigid foam used as insulation (Tricast-5), and a common washing sponge. The flexible foam, which is a thermoplastic PU, achieved a conversion of 79%, and the isolated aniline fraction contained a mixture of 2,4- and 2,6-diaminotoluene in a 3:1 ratio. The PU component of the washing sponge (separated from the polyethylene-based scouring pad) achieved a conversion of 93%, and the isolated aniline fraction was found to contain 2,4- and 2,6-diaminotoluene in a 2:1 ratio. The high-density rigid foam is a thermoset, meaning that it does not soften at elevated temperatures, which poses a challenge for conversion via the hydrogenation pathway. Consequently, only 8% conversion was achieved under the typical conditions using Hz as a reducing agent. Conducting the reaction in EtOH at 160 °C, a conversion of 55% was obtained after 14 h, with the major products being aniline and p-toluidine. Without wishing to be bound by scientific theory, these products are believed to be obtained from the deamination of toluene diamine over a prolonged reaction time. By increasing the reaction temperature to 180 °C, near-quantitative conversion was achieved. These results show that the method according to the invention is well suited to the conversion of commercial PUs.

As elucidated in the given examples, the method according to the invention allows the synthesis of amines from carbamate compounds with good yield, circumventing the disadvantages inherent in the established methods of the prior art.

## Claims

1. Method for manufacturing an amine, in particular an aromatic amine, from a carbamate compound, in particular an aromatic carbamate compound, comprising a cleaving step, in which the aromatic carbamate compound is heated to a temperature of 50 °C to 500 °C in the presence of a reducing agent and in the presence of a catalyst, wherein the catalyst has Lewis acidity and comprises a metal.

2. Method according to claim 1, **characterized in that** the amine is selected from the group consisting of hexamethylenediamine, isophorone diamine, cyclohexylamine, aminotoluene, aniline, ethylaniline, benzyl aniline, diaminotoluene, methylene diphenyl diamine, polymeric methylene diphenyl diamine, tetramethylxylylene diamine, and mixtures thereof, preferably selected from the group consisting of aminotoluene, aniline, ethylaniline, benzyl aniline, diaminotoluene, methylene diphenyl diamine, polymeric methylene diphenyl diamine, tetramethylxylylene diamine, and mixtures thereof.

3. Method according to any one of claims 1 or 2, **characterized in that** the carbamate compound is a non-polymeric compound or a polymeric compound, in particular a polyurethane.

4. Method according to any one of the preceding claims, **characterized in that** the cleaving step is conducted for a period of 15 min to 48 h, preferably 30 min to 24 h, more preferably 45 min to 12 h, most preferably 1 h to 5 h.

5. Method according to any one of the preceding claims, **characterized in that** the metal comprised in the catalyst is selected from the group consisting of aluminum, zirconium, silicon, titanium, niobium, lanthanum, cerium, iron, copper, vanadium, molybdenum, tin, indium, cadmium, rhodium, antimony, germanium, zinc, bismuth, lead, gallium, and mixtures thereof, preferably selected from the group consisting of aluminum, lanthanum, cerium and mixtures thereof, more preferably that the metal comprised in the catalyst is cerium, and/or **in that** the catalyst is a metal oxide.

6. Method according to any one of the preceding claims, **characterized in that** the catalyst is a heterogeneous catalyst.

7. Method according to any one of the preceding claims, **characterized in that** the catalyst is selected from the group consisting of titanium dioxide, zirconium dioxide, niobium pentoxide, aluminum cerium trioxide, lanthanum oxide, cerium dioxide, γ-aluminum oxide, Y-zeolite, ZSM-5 zeolite, vanadium pentoxide, cuprous oxide, molybdenum oxide, stannic oxide, diindium trioxide, cadmium oxide, rhodium oxide, diantimony trioxide, dibismuth trioxide, lead oxide, germanium dioxide, zinc oxide, gallium oxide, and mixtures thereof, preferably selected from the group consisting of aluminum cerium trioxide, lanthanum oxide, cerium dioxide, γ-aluminum oxide, Y-zeolite, and mixtures thereof, more preferably cerium dioxide.

8. Method according to any one of the preceding claims, **characterized in that** the carbamate compound is heated in the cleaving step to a temperature of 80°C to 400°C, preferably 100°C to 300°C, more preferably 150°C to 250°C.

9. Method according to any one of the preceding claims, **characterized in that** the reducing agent is selected from the group consisting of hydrogen, sodium hydride, sodium borohydride, lithium aluminum hydride, hydrazine, formic acid, hydrogen donors such as water, alcohols, in particular methanol, ethanol, isopropanol or glycerol, and saturated alkanes such as polyethylene and mixtures thereof, preferably **in that** the reducing agent is hydrogen, in particular hydrogen at a pressure from 1 to 200 bar, preferably from 1 to 150 bar, more preferably from 5 to 100 bar, even more preferably from 5 to 50 bar, most preferably from 8 to 30 bar.

10. Method according to any one of the preceding claims, **characterized in that** the cleaving step is conducted in the absence of a solvent.

11. Method according to any one of the preceding claims, **characterized in that** the catalyst is present in an amount of 0.01 wt.% to 20 wt.%, preferably 0.1 wt.% to 15 wt.%, more preferably 1 wt.% to 10 wt.%, based on the mass of the carbamate compound.

12. Method according to any one of the preceding claims, **characterized in that** the method comprises an activation step in which the catalyst is heated to a temperature of 300°C or more, preferably from 300°C to 1000°C, more preferably from 400°C to 800°C.

13. Method according to any of the preceding claims, **characterized in that** the method also produces alcohols and/or polyols.
